# EUROPÄISCHE PATENTSCHRIFT

(11) **EP 1 046 636 B1**
(45) Veröffentlichungstag und Bekanntmachung des Hinweises auf die Patenterteilung: **02.01.2004**
(21) Anmeldenummer: 00105351.1
(22) Anmeldetag: 17.03.2000
(51) Int. Cl.: C07C 263/20

(54) **Verfahren zur Verringerung des Chlorgehaltes von organischen Isocyanaten**
Process for the reduction of chlorine content of organic isocyanates
Procédé de réduction de la teneur en chlore d'isocyanates organiques

(30) Priorität: 30.03.1999 DE 19914292
(43) Veröffentlichungstag der Anmeldung: 25.10.2000
(73) Patentinhaber: BAYER AG, 51368 Leverkusen (DE)
(72) Erfinder: Danielmeier, Karsten, Dr., Bethel Park, PA 15102 (US); Leps, Harald, 51373 Leverkusen (DE); Mager, Dieter, 51373 Leverkusen (DE); Halpaap, Reinhard, Dr., 51519 Odenthal (DE); Brahm, Martin Dr., 51519 Odenthal (DE); Klipper, Reinhold Dr., 50933 Köln (DE); Hauner,Andreas Dr., 51373 Leverkusen (DE)

(56) Entgegenhaltungen:
- DD-A- 288 593
- DE-A- 4 232 769
- US-A- 4 996 351

## Beschreibung

Die Erfindung betrifft ein neues Verfahren zur Reinigung von organischen Isocyanaten oder Isocyanatgemischen von Chlorverbindungen durch Vermischen der Isocyanate oder Isocyanatgemische mit gelförmigen oder makroporösen, anionenaustauschenden organischen Materialien mit tertiären und/oder quartären Aminogruppen. Das Verfahren ermöglicht eine mildere Reinigung der Isocyanate von chlorhaltigen Verbindungen als konventionelle Verfahren und eignet sich daher besonders für temperaturempfindliche Isocyanate.

Herstellbedingte Verunreinigungen wechselnder Art und Menge in organischen Isocyanaten sind Anlass für Aktivitätsschwankungen. Solche Aktivitätsschwankungen sind ungünstig für eine reproduzierbare, das heißt wirtschaftliche, Verwendung. So enthalten sowohl aromatische Isocyanate, zum Beispiel die bekannten Phosgenierungsprodukte von Anilin-Formaldehyd-Kondensaten oder auch 2,4- und 2,6-Diisocyanatotoluol, aber auch aliphatische Isocyanate, wie zum Beispiel Isophorondiisocyanat, eine Fülle derartiger Verunreinigungen. Es handelt sich hierbei vor allem um Chlor-enthaltende Verbindungen, die immer dann Aktivitätsschwankungen verursachen, wenn es sich um "leicht bewegliches", sogenanntes hydrolisierbares Chlor handelt. Ein Teil dieser Verbindungen erweist sich als relativ stabil und verbleibt auch nach Destillation in den Isocyanaten. Er beeinflusst neben der Aktivität auch die Stabilität der Isocyanate ungünstig. Ein einheitlicher, niedriger Anteil dieser Verunreinigungen mit der Folge einer Aktivitätsnormierung und leichteren Weiterverarbeitung der Isocyanate ist daher sowohl von technischer wie auch von wirtschaftlicher Bedeutung.

Es hat daher nicht an Versuchen gefehlt, Möglichkeiten zur Entfernung der chlorhaltigen Verbindungen zu finden. In einer Vielzahl von Patentanmeldungen werden thermische Verfahren beschrieben. Beispielsweise ist bekannt, dass Erhitzen von Isocyanaten, insbesondere bei gleichzeitigem Strippen mit Inertgas, oder Erhitzen in inerten Lösungsmitteln unter Druck, bei gleichzeitigem Absaugen der flüchtigen Verbindungen, den Gehalt an leichtspaltbaren Chlorverbindungen vermindert (z. B. DE-A 12 70 036, DD 271 820, US-A 3 219 678, GB-A 1 080 717, DE-A 22 37 552; US-A 3 857 871, US-A 1 458 223, JP 0 727 808 8 A2, JP 0 634 570 7 A2, GB-A 1 384 065).

Aus den Publikationen JP 6 116 125 0 A, JP 0 516 323 1 A, DE-A 19 50 101, DE-A 19 38 384, DE-A 25 32 722, DE-A 26 31 168, US-A 3 853 936, FR-A 1 555 517, DE-A 29 33 601, US-A 3 549 504 ist bekannt, dass Isocyanate durch spezielle Destillations- und Kristallisationstechniken gereinigt werden können.

Eine effektive Abtrennung der störenden Chlorverbindungen ist bei all diesen Verfahren, die auf einer rein physikalischen Behandlung beruhen, nicht gegeben, da hierdurch nur leicht spaltbare Chlorverbindungen abgetrennt werden können. Die Anwendung derartiger Verfahren beschränkt sich so auf spezielle im allgemeinen thermostabile Isocyanatverbindungen, bei deren Einsatz eine eingeschränkte Absenkung des Chlorgehaltes ausreicht.

Neben der rein thermischen Behandlung von Isocyanatverbindungen sind auch Behandlungen mit Zusätzen beschrieben, die eine verbesserte Abtrennung von störenden Chlorverbindungen erlauben. So werden in den Patentschriften JP45010329B, JP 4 200 413 7 B, JP 5 908 845 2 A, JP 5 910 875 3 A, JP 5 917 245 0 A, US-A 3 373 182, GB-A 1 111 581, US-A 3 759 971, US-A 4 094 894, ZA-A 8 100 606, DE-A 11 38 040, DE-A 12 86 025, US-A 3 458 558, US-A 3 264 336, JP 0 105 274 7 A2, SU 8 066 77 und DE-A 22 10 607 auf Metallen oder Alkalimetallen basierte Zusätze beschrieben, wie z. B. Metalloxide, Metallcyanamide, Metallhydride, Metallfettsäureester in Gegenwart von sterisch gehinderten Phenolen, Metallnaphthenate, Metallsilikate, Alkalimetallcarbonate, Organometallverbindungen oder (Alkali)metallhaltige synthetische Zeolithe. Diese Zusätze können jedoch zum Teil schlecht vom gereinigten Isocyanat abgetrennt werden und führen zu einer unerwünschten Metall/Metallionen-Kontamination entsprechender Isocyanatprodukte. Des weiteren bewirken fast alle Metalle und Metallkomplexe eine erhöhte Nebenproduktbildung (Trimerisat-, Carbodiimid-, Dimerbildung).

Ähnliche Beschränkungen findet man bei der Verwendung von Zusätzen wie dem in GB-A 1 347 647 und JP 0 505 898 2 A beschriebenen Imidazol, den in GB-A 1 458 747 beschriebenen Sulfonsäuren und deren Estern, den in GB-A 1 459 691 beschriebenen Diethylsulfat und der auch dort beschriebenen Schwefelsäure sowie der Verwendung von anderen Zusätzen, wie zum Beispiel Epoxyverbindungen (DE-A 22 49 375; JP 0 932 396 8 A2), tetrasubstituierten Harnstoffen (DD 288 598), Ameisen- oder Essigsäure oder deren Derivaten (US-A 3 799 963) oder den in EP-A 524 507 beschriebenen Trimethylsilylgruppen aufweisenden Verbindungen. Der beschriebene Einsatz gelöster Säuren und Basen führt besonders bei der Reinigung von reaktiven Isocyanatverbindungen zu unerwünschten Nebenreaktionen wie Trimerisierung, Dimerenbildung, bzw. Carbodiimidbildung.

Einige Verbindungen mit mindestens einer Zerevitinov-aktiven NH-Gruppe wie Harnstoffe (DD 285 594), Biurete (DD 288 597), Caprolactam (DD 285 593), Epoxide in Gegenwart von Aminen (JP 0 932 396 8 A2), sowie Ammoniumsalze (DD 288 594), Carbodiimide (DD 288 599), Alkylphosphate (DD 288596), tertiäre Alkohole und tertiäre Alkylcarbamate (DD 288 595) werden nach dem Stand der Technik zur Reinigung von Isocyanaten empfohlen. Auch hier ist die Abtrennung der Zusätze bzw. die eingeschränkte Verwendung der zusatzhaltigen Isocyanate und/oder Destillationsrückstände, sowie besonders die teilweise starke Absenkung des NCO-Wertes und die Steigerung in der Viskosität, die sich zum Beispiel auf die Bildung von Biureten bei der Verwendung von tertiären Alkoholen zurückführen lässt, nachteilig. Letzteres gilt insbesondere auch für die Verwendung von Wasser zur Reinigung von Isocyanaten (DE-A 12 40 849).

Die Patentschrift US-A 4 996 351 beschreibt die Verwendung von polymerem stark sauren Material mit pKa Werten ≤ 2 zur Absenkung der Menge an hydrolysierbarem Chlor. Das polymere, stark saure Material wird gemäß der Ausführungsbeispiele zum siedenden Isocyanat zugegeben und senkt dann effektiv den Anteil an hydrolysierbaren Chlor. Anorganische Säuren und stark saure organische Verbindungen sind als effektive Allophanatisierungskatalysatoren beschrieben (z.B. US 4 160 080), wirken stark korrosionsfördernd und sind daher zur Reduktion des HC-Gehaltes von organischen Isocyanaten weniger geeignet.

Die Patentschrift DD 288 593 beschreibt als Zusätze zur Absenkung des Anteils an hydrolysierbarem Chlor die Verwendung von Salzen primärer und sekundärer Amine, die bei Temperaturen von mindestens 200°C unter gleichzeitigem Einsatz von Inertgasen eine effektive Absenkung des hydrolisierbaren Chlorgehaltes erlauben. In den Ausführungsbeispielen sind für eine effektive HC-Absenkung Temperaturen von 225°C als notwendig vorgegeben. Diese hohen Temperaturen beschränken die Anwendung des Verfahrens auf wenige relativ thermostabile Isocyanate. Nach allgemein anerkannter Lehre sind Isocyanatverbindungen, insbesondere temperaturempfindliche niedermolekulare Isocyanate, bei derartig hohen Temperaturen nicht stabil und zersetzen sich beispielsweise unter Bildung von Carbodiimiden und Isocyanuraten. Dieses kann zu einem unkontrollierten Auftreten von Prozesswärme führen, das die Reaktionsführung erheblich erschwert. Weiterhin führt nach der DD 288 593 zwingende Einsatz von Inertgas zwangsläufig zu einem hochbelasteten Abgasstrom mit umwelt- und sicherheitsgefährdendem Potential. Schließlich ist eine Abtrennung der beschriebenen Salze primärer und sekundärer Amine von gereinigten niedermolekularen Isocyanaten mittels Destillation oder Extraktion oder anderer Techniken schwierig, insbesondere auch, weil, wie in der DD 288 593 beschrieben, die unerwünschte Reaktion von Aminsalz mit den zu reinigenden Isocyanaten zu starken Ausbeuteverlusten und Nebenproduktbildung führt.

Die DE-A-42 32 769 offenbart ein Verfahren zur Herstellung von Mischungen aus Diphenylmethan-diisocyanaten und Polyphenyl-polymethylen-polyisocyanaten (Roh-MDI) mit einer verminderten lodfarbzahl durch Umsetzung der entsprechenden Mischungen aus Diphenylmethan-diaminen und Polyphenyl-polymethylen-polyaminen mit Phosgen in Gegenwart mindestens eines inerten organischen Lösungsmittels bei erhöhter Temperatur, wobei man nach beendeter Phosgenierung der Reaktionsmischung Amine, Harnstoffverbindungen oder Mischungen davon in einer wirksamen Menge, zweckmäßigerweise in einer Menge von 0,01 bis 2,5 Gew.-%, bezogen auf das Roh-MDI-Gewicht, einverleibt, danach das Phosgen und das inerte organische Lösungsmittel abtrennt, dem Reaktionsprodukt gegebenenfalls bis 5 Gew.-%, bezogen auf das Roh-MDI-Gewicht, mindestens eines Antioxidans auf Phenolbasis und/oder Arylphosphit hinzufügt und die Reaktionsmischung thermisch behandelt.

Es war daher Aufgabe der vorliegenden Erfindung, ein universell anwendbares Verfahren zur Reinigung von organischen niedermolekularen Isocyanaten insbesondere temperaturempfindlichen niedermolekularen Isocyanaten zur Verfügung zu stellen, welches die angesprochenen Mängel nicht aufweist und eine effiziente Abtrennung des Isocyanates von den verwendeten Reagentien zur Absenkung des Anteils an hydrolysierbaren Chlor ermöglicht.

Diese Aufgabe konnte mit dem nachstehend näher beschriebenen erfindungsgemäßen Verfahren gelöst werden, indem den zu behandelnden organischen Isocyanaten oder Isocyanatgemischen eine geeignete Menge an nachfolgend näher beschriebenem gelförmigem oder makroporösem, anionenaustauschendem organischem Material mit tertiären und/oder quartären Aminogruppen zugesetzt wird und das Gemisch für eine bestimmte Zeit, gegebenenfalls unter erhöhtem oder vermindertem Druck, umgesetzt wird. Überraschender Weise erlaubt das erfindungsgemäße Verfahren eine effektive Abtrennung von störenden Chlorverbindungen (Absenkung des hydrolisierbaren Chlorgehalts) bei Temperaturen von weit unter 200°C ohne zwingenden Einsatz von Inertgas, ohne die Anwendung saurer Bedingungen und ohne die Bildung relevanter Mengen an Nebenprodukten.

Die erfindungsgemäße Anwendung von gelförmigen oder makroporösen, anionenaustauschenden organischen Materialien mit tertiären und/oder quartären Aminogruppen zur effektiven Absenkung der Menge an hydrolysierbaren Chlor war überraschend, da niedermolekulare tertiäre und quartäre Amine bzw. Aminsalze als Trimerisierungskatalyssatoren bekannt sind und somit zur Nebenproduktbildung neigen sollten.

Gegenstand der Erfindung ist ein Verfahren zur Reinigung niedermolekularer Isocyanate von Chlorverbindungen dadurch gekennzeichnet, dass man die niedermolekularen Isocyanate oder Isocyanatgemische
a) mit mindestens 0,1 Gew.-% (bezogen auf Isocyanat) eines tertiäre und/oder quartäre Aminogruppen enthaltenden, gelförmigen oder makroporösen anionenaustauschenden organischen Materials, welches weitgehend frei von Metallionen, Wasser und mit Isocyanaten reagierenden Lösungsmitteln bzw. Beimischungen ist, versetzt,
b) für mindestens 10 Minuten auf
c) eine Temperatur < 200°C erhitzt und im Anschluss
d) gegebenenfalls mittels Filtration und/oder destillativer und/oder extraktiver Aufarbeitung und/oder anderen Trenntechniken von dem gelförmigen oder makroporösen, anionenaustauschenden organischen Material mit tertiären und/oder quartären Aminogruppen und gegebenenfalls entstandenen Reaktionsprodukten befreit.

Ausgangsmaterialien für das erfindungsgemäße Verfahren sind beliebige niedermolekulare Isocyanate, wobei hierunter auch beliebige Gemische von niedermolekularen Isocyanaten verstanden werden. Beispiele für derartige niedermolekulare Isocyanate sind
a) Monoisocyanate mit aliphatisch, cycloaliphatisch, araliphatisch oder aromatisch gebundenen Isocyanatgruppen wie z. B. Butylisocyanat, Stearylisocyanat, Cyclohexylisocyanat, Benzylisocyanat, 2-Phenylethylisocyanat, Phenylisocyanat oder beliebige Gemische solcher Monoisocyanate;
b) Diisocyanate des Molekulargewichtbereichs 140 g/mol bis 400 g/mol mit aliphatisch, cycloaliphatisch, araliphatisch und/oder aromatisch gebundenen Isocyanatgruppen, wie z. B. 1,4-Diisocyanatobutan, 1,6-Diisocyanatohexan (HDI), 2-Methyl-1,5-diisocyanatopentan, 1,5-Diisocyanato-2,2-dimethylpentan, 2,2,4- bzw. 2,4,4-Trimethyl-1,6-diisocyanatohexan, 1,10-Diisocyanatodecan, 1,3- und 1,4-Diisocyanatocyclohexan, 1,3- und 1,4-Bis-(isocyanatomethyl)-cyclohexan, 1-Isocyanato-3,3,5-trimethyl-5-isocyanatomethylcyclohexan (Isophorondiisocyanat, IPDI), 4,4'-Diisocyanatodicyclohexylmethan, 1-Isocyanato-1-methyl-4(3)isocyanatomethylcyclohexan (IMCI), Bis-(isocyanatomethyl)-norbornan, 2-Methylpentan-2,4-diisocyanat, 1,3- und 1,4-Bis-(2-isocyanatoprop-2-yl)-benzol (TMXDI), 2,4- und 2,6-Diisocyanatotoluol (TDI), 2,4'- und 4,4'-Diisocyanatodiphenylmethan, 1,5-Diisocyanatonaphthalin, Dipropylenglycoldiisocyanat, 2,4- oder 2,6-Diisocyanato-1-methylcyclohexan oder beliebige Gemische solcher Diisocyanate,
c) Triisocyanate und/oder höherfunktioneller Isocyanate wie z. B. 4-Isocyanatomethyl-1,8-octandiisocyanat (Nonantriisocyanat), 1,6,11-Undecantriisocyanat, 3-Isocyanatomethyl-1,6-hexamethylendiisocyaynat oder beliebige Gemische solcher Isocyanate.

Ausgangsmaterialien für das erfindungsgemäße Verfahren können selbstverständlich auch beliebige Gemische aus Mono- und/oder Di- und/oder Triisocyanaten und/oder höherfunktionellen Isocyanaten sein.

Allgemein können als niedermolekulare Isocyanate alle organischen Isocyanate eines Molekulargewichts bis ca. 400 g/mol, vorzugsweise von 99 bis 279 g/mol Verwendung finden.

Bevorzugt kommen beim erfindungsgemäßen Verfahren die genannten Di- und höherfunktionellen Isocyanate zum Einsatz. Ganz besonders bevorzugt ist die Verwendung von 4-Isocyanatomethyl-1,8-octandiisocyanat (Nonantriisocyanat).

Das gelförmige oder makroporöse, anionenaustauschende organische Material des erfindungsgemäßen Verfahrens, kann jedes anionenaustauschende organische Material sein, das tertiäre und/oder quartäre Aminogruppen enthält und dadurch gekennzeichnet ist, dass es
a) weitgehend frei von Wasser und anderen mit Isocyanaten reagierenden Lösungsmitteln sowie Beimischungen ist,
b) frei von isocyanatreaktiven funktionellen Gruppen wie Alkoholgruppen, Thiolgruppen, 1°- und 2°-Aminogruppen und Carbonsäuregruppen ist,
c) sich durch gängige Trennmethoden wie Filtration und/oder Extraktion und/oder Destillation von den Isocyanaten abtrennen lässt und
d) weitgehend frei von Metallionen ist.

Bei dem gelförmigen oder makroporösen, anionenaustauschenden organischen Material handelt es sich zum Beispiel um tertiäre und/oder quartäre Aminogruppen enthaltende Materialien, wie sie beispielsweise in *Ullmann* (Bd. A14;S 393 ff), in der DE-A 24 18 976 oder in der DE-A 22 11 134 beschrieben sind und deren Matrix durch Kondensation (z. B. Phenol-Formaldehyd) oder durch Polymerisation (z.B. Copolymere aus Styrol und Divinylbenzol oder (Meth)acrylaten und Divinylbenzol) erhalten werden. Alle genannten Materialien müssen vor der Verwendung im erfindungsgemäßen Verfahren im Sinne des erfindungsgemäßen Verfahren weitgehend von Wasser befreit werden.

Weitgehend frei von Wasser im Sinne des erfindungsgemäßen Verfahrens bedeutet, der Anteil liegt nach Karl-Fischer-Titration bei < 5 %, bevorzugt < 3%, besonders bevorzugt < 1%. Dies kann durch einen Austausch des Wassers gegen ein inertes, nicht mit Isocyanaten reagierendes Lösungsmittel geschehen oder durch Trocknung der anionenaustauschenden Materialien bevorzugt im Vakuum.

Unter weitgehend frei von mit Isocyanaten reagierenden Lösungsmitteln und Beimischungen sowie Metallionen soll im Rahmen des erfindungsgemäßen Verfahrens ein Gehalt an derartigen Verbindungen von unter 5 % vorzugsweise unter 3 % und besonders bevorzugt unter 1 % verstanden werden.

Die im erfindungsgemäßen Verfahren verwendeten anionenaustauschenden Materialien können entweder als Feststoffe ohne Lösungsmittel oder in Form einer Anschwemmung in einem inerten, nicht mit Isocyanaten reagierenden Lösungsmittel verwendet werden.

Zu den im erfindungsgemäßen Verfahren verwendeten Materialien gehören von Wasser und anderen mit Isocyanaten reagierenden Lösungsmitteln befreite Materialien wie zum Beispiel: die Lewatite® MP 62, MP 64, AP 49, M 510, M 511, M 610, MP 500, MP 600, AP 246, S 6328 A, S 5428 A (alle Fa. Bayer AG) oder anionenaustauschende Amberlite® oder Duolite® (beide Fa. Rohm und Haas). Bevorzugte Materialien im Sinne des erfindungsgemäßen Verfahrens sind die oben beschriebenen anionenaustauschenden organischen Materialien deren Matrix durch Polymerisation (Copolymere aus Styrol und Divinylbenzol sowie aus (Meth)acrylaten und Divinylbenzol) erhalten wurden. Besonders bevorzugt sind im erfindungsgemäßen Verfahren anionenaustauschende organische Materialien deren Matrix durch Polymerisation (Copolymere aus Styrol und Divinylbenzol) erhalten wurde und die tertiäre Aminogruppen enthalten.

Unter Material sollen im Sinne des erfindungsgemäßen Verfahrens sowohl definierte Verbindungen, Gemische von Verbindungen, als auch durch statistische Molekulargewichtsverteilung charakterisierte polymere Zusammensetzungen oder Gemische von diesen verstanden werden, die die oben beschriebenen Voraussetzungen erfüllen. Selbstverständlich können auch Gemische von verschiedenen Materialien im erfindungsgemäßen Verfahren verwendet werden.

Die festen oder makroporösen anionenaustauschenden organischen Materialen mit tertiären und/oder quartären Aminogruppen können technische Produkte sein, die selbstverständlich, wenn auch weniger bevorzugt, noch geringe Anteile von freien Aminen bzw. Aminsalzen oder anderen Nebenprodukten enthalten können.

Selbstverständlich können die im erfindungsgemäßen Verfahren verwendeten anionenaustauschenden Materialien durch geeignete Verfahrensschritte regeneriert und nochmals im erfindungsgemäßen Verfahren wiederverwendet werden.

Das vorliegende erfindungsgemäße Verfahren sieht vor, das Gemisch aus organischem Isocyanat und mindestens 0,1 %, vorzugsweise 0,5 bis 50 % und besonders bevorzugt 1,0 bis 10 % bezogen auf Masse Isocyanat des gelförmigen oder makroporösen, anionenaustauschenden organischen Materials mit tertiären und/oder quartären Aminogruppen für mindestens 10 Minuten, vorzugsweise 1 Stunde bis 24 Stunden, besonders bevorzugt 3 Stunden bis 15 Stunden bei einer Temperatur von <200°C, vorzugsweise 25 bis 200°C, besonders bevorzugt 140°C bis 190°C und ganz besonders bevorzugt 150°C bis 180°C zu erhitzen und gegebenenfalls im Anschluss das gereinigte, niedermolekulare Isocyanat von dem gelförmigen oder makroporösen, anionenaustauschenden organischen Material mit tertiären und/oder quartären Gruppen und gegebenenfalls entstehenden Reaktionsprodukten durch Filtration und/oder destillativ und/oder extraktiv und/oder durch andere geeignete Trennmethoden zu entfernen.

Selbstverständlich kann die Umsetzung auch in einem geeigneten inerten, nicht mit Isocyanaten reaktiven Lösungsmittel durchgeführt werden

Das Isocyanat kann während oder nach der Temperung durch Filtration und/oder extraktiv und/oder destillativ und/oder durch andere geeignete Trennmethoden, gegebenenfalls im Vakuum, von den gelförmigen oder makroporösen, anionenaustauschenden organischen Materialen mit tertiären und quartären Aminogruppen und gegebenenfalls entstehenden Reaktionsprodukten getrennt werden. Vorzugsweise wird das gereinigte Isocyanat durch Filtration von den gelförmigen oder makroporösen anionenaustauschenden organischen Materialien mit tertiären und/oder quartären Aminogruppen und/oder dessen Reaktionsprodukten getrennt.

Zur Vermeidung von Nebenreaktionen mit Luft bzw. Spuren von Wasser kann die Reaktionsapparatur unter Vakuum (beispielsweise 100 bis 1 mbar) betrieben werden oder eine Beschleierung mit Inertgas, beispielsweise Stickstoff, erfolgen. Prinzipiell ist der Einsatz von Inertgas für das erfindungsgemäße Verfahren jedoch nicht zwingend notwendig.

Erfindungsgemäß ist es selbstverständlich auch möglich, in Art einer Säulentechnik eine Temperung der Isocyanatverbindungen mit den beschriebenen anionenaustauschenden Materialien durchzuführen, indem das anionenaustauschende organischen Material als stationäre Phase dient und die entsprechende Isocyanatverbindung bei vorgegebenen Bedingungen hindurchfließt. Die Temperzeit ist hierbei mit der mittleren Verweilzeit der Isocyanatverbindung im anionenaustauschenden organischen Material gleichzusetzen.

Vor, während oder nach der Absenkung des Chlorgehaltes nach dem erfindungsgemäßen Verfahren können auch noch andere Reinigungsmethoden Verwendung finden, um beispielsweise farbgebende Komponenten und Nebenprodukte zu entfernen. Hierzu zählen unter anderem Behandlungen und/oder Aufhellungen beispielsweise mit Reduktions- oder Oxidationsmitteln und die Behandlung mit Adsorpionsmitteln wie Aktivkohle und/oder Bleicherden und/oder Kieselsäuren. Derartige Aufhellungen können ebenfalls einen positiven Effekt auf die Absenkung des Chlorgehalts der Isocyanatverbindung ausüben. Das nach der Absenkung des Chlorgehaltes und von dem gelförmigen oder makroporösen, anionenaustauschenden organischen Materialien mit tertiären und/oder quartären Aminogruppen befreite, vorliegende Isocyanat kann zudem optional vorzugsweise unter Vakuum (z. B. 0.001 mbar bis 100 mbar) durch Destillation (zum Beispiel Blasendestillation oder Destillation mittels eines Dünnschichtverdampfers) einer weiteren Aufreinigung unterzogen werden.

Erfindungsgemäß gereinigte Isocyanate weisen keine schädlichen Zusätze von Metallverbindungen, Säuren, Basen oder anderen isocyanatreaktiven Verbindungen auf und haben einen HC-Gehalt von vorzugsweise < 180 ppm. Sie können breit eingesetzt werden z. B. zur Herstellung von oligomeren Polyisocyanaten oder Prepolymeren und im Falle der Triisocyanate als Ausgangsmaterialien für Zwischenprodukte, Polyurethanformteile und Beschichtungsmittel. Vorzugsweise werden die nach dem erfindungsgemäßen Verfahren gereinigten niedermolekularen Triisocyanate als Härterkomponente in Beschichtungen eingesetzt.

Prinzipiell eignen sich Beschichtungsmaterialien, die nach dem erfindungsgemäßen Verfahren gereinigte Isocyanate enthalten, zur Beschichtung beliebiger Substrate wie beispielsweise Holz, Kunststoffe, Leder, Papier, Textilien, Glas, Keramik, Putz, Mauerwerk, Metalle oder Beton. Sie lassen sich mit üblichen Applikationsmethoden wie Spritzen, Streichen, Fluten, Gießen, Tauchen, Walzen aufbringen. Die Beschichtungsmaterialien können in Form von Klarlacken als auch in Form pigmentierter Lacke verwendet werden, wobei sie verdünnt in organischen Lösemitteln bzw. dispergiert in Wasser oder unverdünnt als Ein- oder Mehrkomponentenbeschichtung zum Einsatz gelangen.

### Beispiele

Die folgenden Ausführungsbeispiele erläutern die Erfindung. Die angegebenen HC-Werte beziehen sich auf den Gehalt an hydrolisierbarem Chlor. Alle Prozentangaben beziehen sich auf das Gewicht.

100 g des organischen Isocyanates werden mit der in Tabelle 1 bzw. 2 angegebenen Menge der Verbindung A ,B oder C bzw. des Gemisches aus A und C versetzt und unter Vakuum (10 - 100 mbar; je nach eingesetztem Isocyanat) für die angegebene Zeitspanne und Temperatur gerührt. Nach der angegebenen Reaktionszeit werden die Produkte einer Filtration (Ausführungsbeispiele 1, 2, 3, 6 und 7) bzw. einer Filtration mit anschließender dünnschichtdestillativen Aufarbeitung (180°C/0,2 mbar) (Ausführungsbeispiele 4, 5) unterzogen. Man erhält die erfindungsgemäßen Produkte.

**Tabelle 1**

| | Vergleichsbeispiel 1 | Vergleichsbeispiel 2 Entsprechend DD 288 593 1. Beispiel | erfindungsgemäßes Beispiel 1 | erfindungsgemäßes Beispiel 2 | erfindungsgemäßes Beispiel 3 |
|---|---|---|---|---|---|
| Organisches Isocyanat | Triisocyanatononan (TIN) | Triisocyanatononan (TIN) | Triisocyanatononan (TIN) | Triisocyanatononan (TIN) | Triisocyanatononan (TIN) |
| Verwendeter Zusatz | - | Dibutylammoniumacetat | A | A | B |
| Gew.-% des Zusatzes | - | 0,12 | 2,5 | 4,5 | 4,5 |
| Temperatur | 170°C | 225°C | 170°C | 170°C | 170°C |
| Reaktionszeit [h] | 12 | 3 | 12 | 12 | 12 |
| HC-Wert vor Konditionierung [ppm] | 2064 | 2064 | 2064 | 2064 | 2064 |
| HC-Wert nach Konditionierung [ppm] | 1445 | 723 | 170 | 116 | 100 |
| NCO vor Temperung [%] | 49,7 | 49,7 | 49,7 | 49,7 | 49,7 |
| NCO nach Temperung [%] | 47,8 | 35,8 | 47,6 | 47,4 | 47,2 |

Verbindung A: Lewatit® MP 62 (Fa. Bayer AG), Austausch des Wassers gegen niedrigsiedende Lösungsmittel und anschließende Trocknung bei 25°C im Hochvakuum.
Verbindung B: Lewatit® MP 64 (Fa. Bayer AG), Austausch des Wassers gegen niedrigsiedende Lösungsmittel und anschließende Trocknung bei 25°C im Hochvakuum.
Verbindung C: Lewatit® M 610 (Fa. Bayer AG), Beladung des Lewatits mit Base; Auswaschen des Natriumchlorids, dann Austausch des Wassers gegen niedrigsiedende Lösungsmittel und anschließende Trocknung bei 25°C im Hochvakuum.

**Tabelle 2**

| | erfindungsgemäßes Beispiel 4 | erfmdungsgemäßes Beispiel 5 | erfmdungsgemäßes Beispiel 6 | erfindungsgemäßes Beispiel 7 |
|---|---|---|---|---|
| Organisches Isocyanat | Triisocyanatononan (TIN) | Triisocyanatononan (TIN) | Isophoron-diisocyanat (IPDI) | Hexamethylen-diisocyanat (HDI) |
| Verwendeter Zusatz | C | A & C | B | B |
| Gew.-% des Zusatzes | 2,5 | 2,5+1,5 | 2,5 | 2,5 |
| Temperatur | 170°C | 170°C | 170°C | 170°C |
| Reaktionszeit [h] | 12 | 12 | 12 | 12 |
| HC-Wert vor Konditionierung [ppm] | 2064 | 2064 | 152 | 38 |
| HC-Wert nach Konditionierung [ppm] | 160 | 175 | 8 | 26 |
| NCO vor Temperung [%] | 49,7 | 49,7 | 38,0 | 49,9 |
| NCO nach Temperung [%] | 46,4 | 46,8 | 35,6 | 47,5 |

Der Unterschied des NCO-Gehalts vor und nach der Temperung gibt Auskunft über unerwünschte Nebenreaktionen und somit den Ausbeuteverlust.

Im nicht erfindungsgemäßen Vergleichsbeispiel 2 werden die Bedingungen der Patentschrift DD 288 593 nachgestellt, um den HC-Gehalt von TIN abzusenken. Die deutliche NCO-Abnahme macht auf eine nicht akzeptabel hohe Belastung aufmerksam.

Beispiel 3 (erfindungsgemäßes Verfahren): Schon bei 170 °C kann der HC-Gehalt bei schonenden Bedingungen auf 100 ppm abgesenkt werden, wobei nur eine geringe NCO-Abnahme festzustellen ist.

## Patentansprüche

1. Verfahren zur Reinigung niedermolekularer Isocyanate von Chlorverbindungen **dadurch gekennzeichnet, dass** man die niedermolekularen Isocyanate oder Isocyanatgemische
a) mit mindestens 0,1 Gew.-% (bezogen auf Isocyanat) eines tertiäre und/oder quartäre Aminogruppen enthaltenden, gelförmigen oder makroporösen anionenaustauschenden organischen Materials, welches weitgehend frei von Metallionen, Wasser und mit Isocyanaten reagierenden Lösungsmitteln bzw. Beimischungen ist, versetzt,
b) für mindestens 10 Minuten auf
c) eine Temperatur von <200 °C erhitzt und im Anschluss
d) gegebenenfalls mittels Filtration und/oder destillativer und/oder extraktiver Aufarbeitung und/oder anderen Trenntechniken von dem gelförmigen oder makroporösen, anionenaustauschenden organischen Material mit tertiären und/oder quartären Aminogruppen und gegebenenfalls entstandenen Reaktionsprodukten befreit.

2. Verfahren gemäß Anspruch 1, **dadurch gekennzeichnet, dass** man die niedermolekularen Isocyanate
a) mit 1,0 bis 10,0 Gew.-% (bezogen auf Isocyanat) eines gelförmigen oder makroporösen, anionenaustauschenden organischen Material mit tertiären und/oder quartären Aminogruppen versetzt für 3 Stunden bis 15 Stunden auf
b) eine Temperatur von 150 bis 180°C erhitzt und im Anschluss
c) mittels Filtration von dem gelförmigen oder makroporösen, anionenaustauschenden organischen Material mit tertiären und/oder quartären Aminogruppen und gegebenenfalls entstandenen Reaktionsprodukten befreit.

3. Verfahren gemäß Anspruch 1 und 2, **dadurch gekennzeichnet, dass** man als Isocyanat 4-Isocyanatomethyl-1,8-octandiisocyanat (Nonantriisocyanat) verwendet.

4. Verfahren gemäß Anspruch 1 und 2, **dadurch gekennzeichnet, dass** die so gereinigten Isocyanate einen Gehalt an hydrolysierbaren Chlor von <180 ppm aufweisen.

## Claims

1. Process for purifying low molecular weight isocyanates by lowering their chlorine compound content, **characterized in that** the low molecular weight isocyanates or isocyanate mixtures
a) are admixed with at least 0.1 wt. % (based on isocyanate) of a gel-type or macroporous anion-exchanging organic material containing tertiary and/or quaternary amino groups which is substantially free of metal ions, water and isocyanate-reactive solvents and admixtures,
b) heated for at least 10 minutes at
c) a temperature of < 200°C and subsequently
d) freed from the gel-type or macroporous anion-exchanging organic material containing tertiary and/or quaternary amino groups and from any reaction products formed, where appropriate, by means of filtration and/or distillative and/or extractive workup and/or other separating techniques.

2. Process according to Claim 1, **characterized in that** the low molecular weight isocyanates
a) admixed with from 1.0 to 10.0 wt. % (based on isocyanate) of a gel-type or macroporous anion-exchanging organic material containing tertiary and/or quaternary amino groups are heated for from 3 hours to 15 hours at
b) a temperature of from 150 to 180°C and subsequently
c) are freed by means of filtration from the gel-type or macroporous anion-exchanging organic material containing tertiary and/or quaternary amino groups and from any reaction products formed.

3. Process according to Claim 1 and 2, **characterized in that** 4-isocyanatomethyl-1,8-octane diisocyanate (nonane triisocyanate) is used as isocyanate.

4. Process according to Claim 1 and 2, **characterized in that** the isocyanates thus purified have a hydrolyzable chlorine content of < 180 ppm.

## Revendications

1. Procédé pour purifier des isocyanates à bas poids moléculaire par élimination des dérivés chlorés **caractérisé en ce que**
a) on ajoute aux isocyanates ou mélanges d'isocyanates à bas poids moléculaire au moins 0,1 % de leur poids d'une matière organique gélifiée ou macroporeuse échangeuse d'anions, à groupes amino tertiaires et/ou quaternaires, qui est pratiquement exempte d'ions métalliques, d'eau, de solvant réagissant avec les isocyanates ou leurs mélanges,
b) on chauffe pendant au moins 10 min,
c) à une température inférieure à 200°C puis
d) le cas échéant, on débarrasse les isocyanates ou mélanges d'isocyanates à bas poids moléculaire de la matière organique gélifiée ou macroporeuse échangeuse d'anions à groupes amino tertiaires et/ou quaternaires et le cas échéant des produits de réaction formés par filtration et/ou distillation et/ou extraction et/ou par d'autres techniques de séparation.

2. Procédé selon la revendication 1, **caractérisé en ce que**
a) on ajoute aux isocyanates à bas poids moléculaire 1,0 à 10,0 % de leur poids d'une matière organique gélifiée ou macroporeuse échangeuse d'anions à groupes amino tertiaires et/ou quaternaires et on chauffe pendant 3 h à 15 h
b) à une température de 150 à 180°C puis
c) on débarrasse les isocyanates à bas poids moléculaire de la matière organique gélifiée ou macroporeuse échangeuse d'anions à groupes amino tertiaires et/ou quaternaires et le cas échéant des produits de réaction formés par filtration.

3. Procédé selon les revendications 1 et 2, **caractérisé en ce que** l'isocyanate traité est le 4-isocyanatométhyl-1,8-octanediisocyanate (nonanetriisocyanate).

4. Procédé selon les revendications 1 et 2, **caractérisé en ce que** les isocyanates purifiés ont une teneur en chlore hydrolysable inférieure à 180 ppm.
